# FASCICULE DE BREVET EUROPEEN

(11) **EP 0 811 371 B1**
(45) Date de publication et mention de la délivrance du brevet: **12.03.2003**
(21) Numéro de dépôt: 97400833.6
(22) Date de dépôt: 11.04.1997
(51) Int. Cl.: A61K 7/06

(54) **Compositions cosmétiques détergentes à usage capillaire et utilisation**
Kosmetisches Haarwaschmittel und dessen Verwendung
Washing hair care composition and use

(30) Priorité: 06.05.1996 FR 9605644
(43) Date de publication de la demande: 10.12.1997
(73) Titulaire: L'OREAL, 75008 Paris (FR)
(72) Inventeur: Decoster, Sandrine, 93800 Epinay sur Seine (FR); Beauquey, Bernard, 92110 Clichy (FR)
(74) Mandataire: Andral, Christophe André Louis

(56) Documents cités:
- EP-A- 0 392 320
- EP-A- 0 433 946
- EP-A- 0 574 156
- EP-A- 0 729 742
- WO-A-92/04882
- WO-A-93/08787
- GB-A- 2 165 550
- DATABASE WPI Section Ch, Week 8927 Derwent Publications Ltd., London, GB; Class A96, AN 89-195612 XP002023233 & JP 01 132509 A (LION CORP), 25 mai 1989

## Description

La présente invention concerne de nouvelles compositions cosmétiques à propriétés améliorées destinées simultanément au nettoyage et au conditionnement des cheveux, et comprenant, dans un support cosmétiquement acceptable, une base lavante constituée de tensioactifs à pouvoir détergent dans laquelle sont également présents à titre d'agents conditionneurs, des polymères cationiques en association avec des silicones particulières. L'invention concerne aussi l'utilisation desdites compositions dans l'application cosmétique susmentionnée.

Pour le nettoyage et/ou le lavage des cheveux, l'utilisation de compositions capillaires détergentes (ou shampooings) à base essentiellement d'agents tensioactifs classiques de type notamment anioniques, non-ioniques et/ou amphotères, mais plus particulièrement de type anioniques, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage nettement accrus par rapport à ce qui peut être obtenu avec les compositions nettoyantes correspondantes qui en sont exemptes. En outre, sur des cheveux sensibilisés, il est connu d'utiliser de préférence un mélange de silicone et de polymère cationique.

Toutefois, et malgré les progrès réalisés récemment dans le domaine des shampooings à base de polymères cationiques et de silicone, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoqués ci-avant, de meilleures performances.

La présente invention vise à la satisfaction d'un tel besoin.

Ainsi, à la suite d'importantes recherches menées sur la question, il a maintenant été trouvé par la Demanderesse, de façon totalement inattendue et surprenante, qu'en utilisant une association de deux types de silicones particulières et convenablement sélectionnées, telles que définies ci-après, dans des compositions capillaires détergentes contenant des polymères cationiques classiques à titre d'agents conditionneurs, il est possible d'améliorer de manière substantielle et significative les propriétés cosmétiques attachées à ces dernières, et ceci tout en conservant leur bon pouvoir lavant intrinsèque.

Les compositions conformes à l'invention confèrent aux cheveux, après rinçage, un remarquable effet traitant qui se manifeste notamment par une facilité de démêlage, ainsi qu'un apport de volume, de légèreté, de lissage, de douceur et de souplesse.

Toutes ces découvertes sont à la base de la présente invention.

Ainsi, selon la présente invention, il est maintenant proposé de nouvelles compositions capillaires détergentes et conditionnantes comprenant, dans un milieu cosmétiquement acceptable, (A) une base lavante et (B) un système conditionneur comprenant au moins un polymère cationique et un mélange d'au moins une silicone aminée et d'au moins une silicone insoluble de viscosité comprise entre 20 et 80 Pa.s.

L'invention a également pour objet l'utilisation en cosmétique des compositions ci-dessus pour le nettoyage et le conditionnement des cheveux.

Mais d'autres caractéristiques, aspects et avantages de l'invention apparaîtront encore plus clairement à la lecture de la description qui va suivre ainsi que des exemples concrets, mais nullement limitatifs, destinés à l'illustrer.

Comme indiqué précédemment, les éléments essentiels rentrant dans la composition des produits capillaires selon l'invention sont (A) une base lavante, (B) un système conditionneur comprenant (i) le ou les polymères cationiques, (ii) la ou les silicones aminées et (iii) la ou les silicones insolubles de viscosité spécifique.

### A- BASE LAVANTE :

Les compositions conformes à l'invention comprennent nécessairement une base lavante, généralement aqueuse.

Le ou les tensioactifs formant la base lavante peuvent être indifféremment choisis, seuls ou en mélanges, au sein des tensioactifs anioniques, amphotères, non ioniques et cationiques.

Toutefois, selon l'invention, la base lavante comprend de préférence des tensioactifs anioniques ou des mélanges de tensioactifs anioniques et de tensioactifs amphotères, et encore plus préférentiellement ne contient que ce type de tensioactif ou mélange de tensioactifs.

La quantité minimale de base lavante est celle juste suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la base lavante peut représenter de 4 % à 50 % en poids, de préférence de 10 % à 35 % en poids, et encore plus préférentiellement de 12 % à 25 % en poids, du poids total de la composition finale.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante : 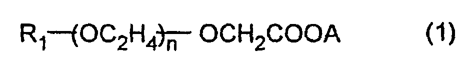 dans laquelle :
R₁ désigne un groupement alkyle ou alkaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Parmi les tensioactifs anioniques, on préfère utiliser selon l'invention les sels d'alkylsulfates et d'alkyléthersulfates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂-CONHCH₂CH₂-N(R₃)(R₄)(CH₂COO⁻) (2)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C) (3)

dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂- CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphocarboxyglycinate vendu sous la dénomination commerciale MIRANOL C2M concentré par la Société MIRANOL.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

On notera que les tensioactifs cationiques, dont l'utilisation n'est pas exclue, ne constituent pas des tensioactifs préférés pour la mise en oeuvre de la présente invention.

### B- SYSTEME CONDITIONNEUR

### (i) Polymère(s) cationique(s) :

Les compositions conformes à l'invention comprennent en outre nécessairement un polymère cationique.

Les agents conditionneurs de type polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire en nombre comprise entre 500 et 5.10⁶ environ, et de préférence comprise entre 10³ et 3.10⁶ environ.

Parmi les polymères cationiques, on peut citer plus particulièrement les protéines (ou hydrolysats de protéines) quatemisées et les polymères du type polyamine, polyaminoamide et polyammonium quaternaire. Ce sont des produits connus.

Les protéines ou hydrolysats de protéines quatemisés sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate";
- les hydrolysats de collagène portant des groupements chlorure de triméthylammonium et de triméthylstéarylammonium, vendus sous la dénomination de "Quat-Pro S" par la Société Maybroook et dénommés dans le dictionnaire CTFA "Steartrimonium Hydrolyzed Collagen" ;
- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :
- le "Croquat L" dont les groupements ammonium quaternaires comportent un groupement alkyle en C₁₂ ;
- le "Croquat M" dont les groupements ammonium quatemairees comportent des groupements alkyle en C₁₀-C₁₈ ;
- le "Croquat S" dont les groupements ammonium quaternaires comportent un groupement alkyle en C₁₈ ;
- le "Crotein Q" dont les groupements ammonium quaternaires comportent au moins un groupe alkyle ayant de 1 à 18 atomes de carbone.

Ces différents produits sont vendus par la Société Croda.

D'autre protéines ou hydrolysats quaternisés sont par exemple ceux répondant à la formule : 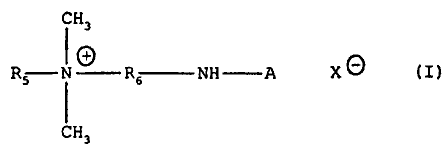 dans laquelle X⁻ est un anion d'un acide organique ou minéral, A désigne un reste de protéine dérivé d'hydrolysats de protéine de collagène, R₅ désigne un groupement lipophile comportant jusqu'à 30 atomes de carbone, R₆ représente un groupement alkylène ayant 1 à 6 atomes de carbone. On peut citer par exemple les produits vendus par la Société Inolex, sous la dénomination "Lexein QX 3000", appelé dans le dictionnaire CTFA (4^{ème} Ed.1991) "Quaternium-76 Hydrolysed Collagen").

On peut encore citer les protéines végétales quaternisées telles que les protéines de blé, de maïs ou de soja : comme protéines de blé quaternisées, on peut citer celles commercialisées par la Société Croda sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Les polymères du type polyamine, polyaminoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) Les copolymères vinylpyrrolidone-acrylate ou -méthacrylate de dialkylamino-alkyle quaternisés ou non, tels que les produits vendus sous la dénomination "Gafquat" par la Société ISP, comme par exemple Gafquat 734, 755 ou HS100 ou bien le produit dénommé "Copolymère 937". Ces polymères sont décrits en détail dans les brevets français 2 077 143 et 2 393 573.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyldiallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-dialcoylaminohydroxyalcoyldialcoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyldiéthylène triamine vendus sous la dénomination "Cartaretine F, F4 ou F8" par la société Sandoz.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipiquelépoxypropylldiéthylène-triamine.
(9) les cyclohomopolymères de méthyl diallyl amine ou de diméthyl diallyl ammonium tels que les homopolymères comportant comme constituant principal de la chaîne des motifs répondant aux formules (VI) ou (VI') : 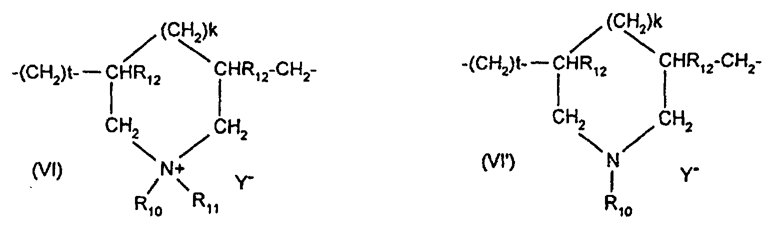 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Merck.
(10) le polymère de diammonium quaternaire contenant des motifs récurants répondant à la formule : 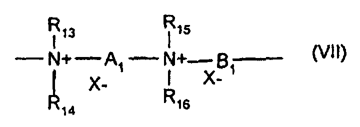 formule (VII) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement (CH₂)n-CO-D-OC-(CH₂)n-
      dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- ;

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) les polymères de polyammonium quaternaires constitués de motifs de formule (VIII): 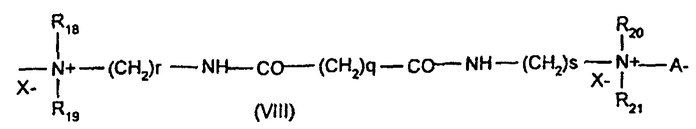 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)pOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A désigne un radical d'un dihalogénure ou représente de préférence -CH₂-CH₂-O-CH₂-CH₂-.

   De tels composés sont notamment décrits dans la demande de brevet EP-A-122 324.
   On peut par exemple citer parmi ceux-ci, les produits "Mirapol A 15", "Mirapol AD1", "Mirapol AZ1" et "Mirapol 175" vendus par la société Miranol.
   (12) les homopolymères ou copolymères dérivés des acides acrylique ou méthacrylique et comportant des motifs : 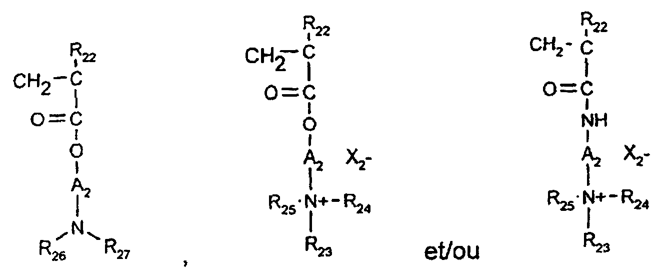 dans lesquels les groupements R₂₂ désignent indépendamment H ou CH₃,
   les groupements A₁ désignent indépendamment un groupe alcoyle linéaire ou ramifié de 1 à 6 atomes de carbone ou un groupe hydroxyalcoyle de 1 à 4 atomes de carbone,
   les groupements R₂₃, R₂₄, R₂₅, identiques ou différents, désignant indépendamment un groupe alcoyle de 1 à 18 atomes de carbone ou un radical benzyle,
   les groupements R₂₆ et R₂₇ représentent un atome d'hydrogène ou un groupement alcoyle de 1 à 6 atomes de carbone,
   X₂⁻ désigne un anion, par exemple méthosulfate ou halogénure, tel que chlorure ou bromure.

   Le ou les comonomères utilisables dans la préparation des copolymères correspondants appartiennent à la famille des acrylamides, méthacrylamides, diacétone acrylamides, acrylamides et méthacrylamides substitués à l'azote par des alcoyle inférieurs, des esters d'alcoyles, des acides acrylique ou méthacrylique, la vinylpyrrolidone ou des esters vinyliques.
(13) Les polymères quaternaires de vinylpyrrolidone et de vinylimidazole tels que par exemple les produits commercialisés sous les dénominations Luviquat FC 905, FC 550 et FC 370 par la société B.A.S.F..
(14) Les polyamines comme le Polyquart H vendu par HENKEL, référencé sous le nom de « POLYETHYLENEGLYCOL (15) TALLOW POLYAMINE » dans le dictionnaire CTFA.
(15) Les polymères réticulés de chlorure de méthacryloyloxyéthyltriméthylammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quatemisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale. Cette dispersion est commercialisée sous le nom de « SALCARE SC 95 » par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Selon l'invention, on peut utiliser plus particulièrement les polymères choisis parmi le Mirapol, le composé de formule (VII) dans laquelle R13, R14, R15 et R16 représentent le radical méthyle, A1 représente le radical de formule -(CH2)3- et B1 représente le radical de formule -(CH2)6- et X' représente l'anion chlorure et le composé de formule (VII) dans laquelle R13 et R14 représentent le radical éthyle, R15 et R16 représentent le radical méthyle, A1 et B1 représentent le radical de formule -(CH2)3- et X⁻ représente l'anion bromure.

Parmi tous. les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société UNION CARBIDE CORPORATION, les cyclopolymères, en particulier les copolymères du chlorure de diméthyldiallylammonium et d'acrylamide, vendus sous les dénominations « MERQUAT 550 » et « MERQUAT S » par la Société MERCK, les polysaccharides cationiques et plus particulièrement la gomme de guar modifiée par du chlorure de 2,3-époxypropyl triméthylammonium vendue sous la dénomination « JAGUAR C13S » par la Société MEYHALL.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

### (ii)- Mélange de silicones

Selon une caractéristique essentielle des compositions capillaires détergentes conformes à l'invention, ces dernières contiennent en outre un mélange d'au moins une silicone aminée et d'au moins une silicone spécifique (différente de la précédente) insoluble. Selon une caractéristique préférée des compositions selon l'invention, le système conditionneur attaché à ces dernières ne contient pas de silicones autres que les silicones aminées et les silicones insolubles de viscosité conforme à l'invention.

### (1)- Silicone(s) aminée(s)

Selon l'invention, on désigne par silicone aminée toute silicone comportant au moins une amine primaire, secondaire, tertiaire ou un groupement ammonium quaternaire. On peut ainsi citer :
(a) les polysiloxanes dénommés dans le dictionnaire CTFA "amodiméthicone" et répondant à la formule : 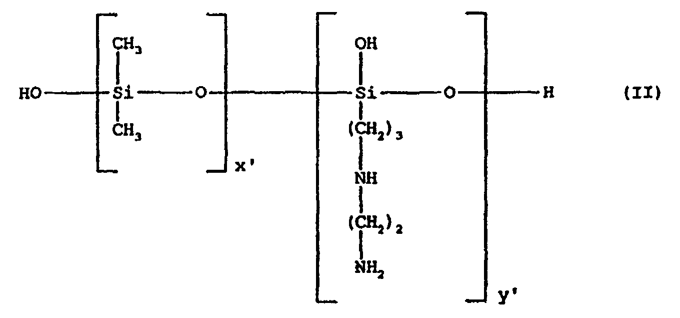 dans laquelle x' et y' sont des nombres entiers dépendant du poids moléculaire, généralement tels que ledit poids moléculaire en nombre est compris entre 5 000 et 500 000 environ ;
(b) les polymères cationiques siliconés répondant à la formule :

   R'ₐG₃₋ₐ-Si(OSiG₂)ₙ-(OSiG_{b}R'_{2-b})ₘ-O-SiG₃₋ₐ-R'ₐ (III)

   dans laquelle :
   G est un atome d'hydrogène, ou un groupement phényle, OH, ou alkyle en C₁-C₈, par exemple méthyle,
   a désigne le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R' est un radical monovalent de formule -C_{q}H_{2q}L dans laquelle q est un nombre de 2 à 8 et L est un groupement aminé éventuellement quatemisé choisi parmi les groupements :

      -NR"-CH₂-CH₂-N'(R")₂

      -N(R")₂

      -N^{⊕}(R")₃ A⁻

      -N^{⊕}(R")₂ HA⁻

      -N^{⊕}H₂(R")A⁻

      -N(R")-CH₂-CH₂-N^{⊕}R" H₂ A⁻,
   dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.

Un produit correspondant à cette définition est le polymère dénommé "triméthylsilylamodiméthicone", répondant à la formule : 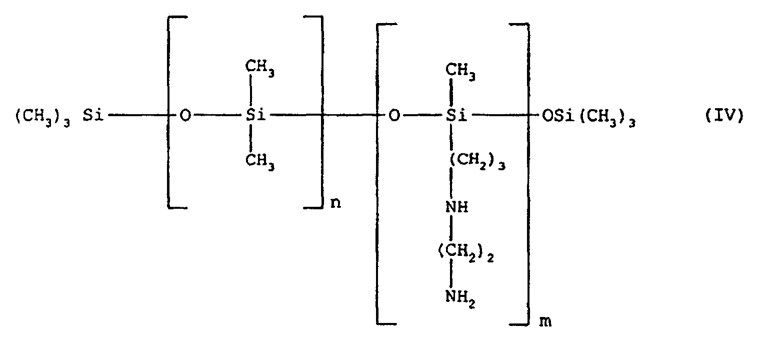 dans laquelle n et m ont les significations données ci-dessus (cf formule III).

De tels polymères sont décrits par exemple dans la demande de brevet EP-A-95238.
(c) les polymères cationiques siliconés répondant à la formule : 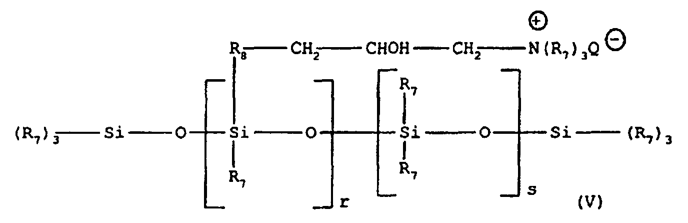
dans laquelle
R₇ représente un radical hydrocarboné monovalent ayant de 1 à 18 atomes de carbone, et en particulier un radical alkyle en C₁-C₁₈, ou alcényle en C₂-C₁₈, par exemple méthyle
R₈ représente un radical hydrocarboné divalent, notamment un radical alkylène en C₁-C₁₈ ou un radical alkylèneoxy divalent en C₁-C₁₈, par exemple en C₁-C₈ ;
Q⁻ est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

De tels polymères sont décrits plus particulièrement dans le brevet US 4 185 087.

Un polymère entrant dans cette classe est le polymère vendu par la Société Union Carbide sous la dénomination "Ucar Silicone ALE 563".

Lorsque ces polymères siliconés sont mis en oeuvre, une forme de réalisation particulièrement intéressante est leur utilisation conjointe avec des agents de surface cationiques et/ou non ioniques. On peut utiliser par exemple le produit vendu sous la dénomination "Emulsion Cationique DC 929" par la Société Dow Coming qui comprend, outre l'amodiméthicone, un agent de surface cationique comprenant un mélange de produits répondant à la formule 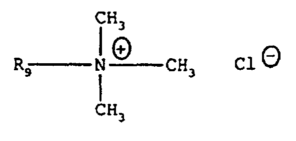 dans lequel R₉ désigne des radicaux alcényle et/ou alcoyle ayant de 14 à 22 atomes de carbone, dérivés des acides gras du suif,
en association avec un agent de surface non ionique de formule :

C₉H₁₉-C₆H₄-(OC₂H₄)₁₀-OH

connu sous la dénomination "Nonoxynol 10".

On peut également utiliser par exemple le produit vendu sous la dénomination "Emulsion Cationique DC 939" par la Société Dow Corning qui comprend, outre l'amodiméthicone, un agent de surface cationique le chlorure de triméthyl cétyl ammonium en association avec un agent de surface non ionique le tridéceth-12.

Un autre produit commercial utilisable selon l'invention est le produit vendu sous la dénomination "Dow Corning Q2 7224" par la Société Dow Coming comportant en association le triméthylsilylamodiméthicone de formule (IV), un agent de surface non ionique de formule : C₈H₁₇-C₆H₄-(OCH₂CH₂)ₙ-OH où n = 40 dénommé encore octoxynol-40,
un autre agent de surface non ionique de formule : C₁₂H₂₅-(OCH₂-CH₂)ₙ-OH où n = 6 encore dénommé isolaureth-6, et du glycol.

Les compositions capillaires conformes à l'invention renferment les silicones aminées définies ci-dessus à des teneurs pondérales qui peuvent être comprises entre 0,05 % et 10 %, de préférence entre 0,1 % et 5 % et encore plus préférentiellement entre 0,2 % et 3%, par rapport au poids total de la composition.

### (2)- Silicone(s) insoluble(s):

La viscosité de ces silicones insolubles est entre 20 et 80 Pa.s.

La viscosité de ces silicones est mesurée à 25°C selon la norme ASTM 445 Appendice C.

Selon la présente invention, par insoluble on entend insoluble dans la composition finale.

Dans tout ce qui suit ou qui précède, on entend désigner par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₁₀ et en particulier méthyle, les radicaux fluoroalkyles, les radicaux aryles et en particulier phényle.

Selon l'invention, la silicone de viscosité convenable est plus particulièrement choisie parmi :
(i) les polydialkylsiloxanes ;
(ii) les polydiarylsiloxanes ;
(iii) les polyalkylarylsiloxanes ;

Parmi les polydialkylsiloxanes, on peut citer principalement :
- les polydiméthylsiloxanes linéaires à groupements terminaux triméthylsilyle, comme par exemple, et à titre non limitatif, les huiles SILBIONE de la série 70047 commercialisées par RHONE POULENC,
- les polydiméthylsiloxanes linéaires à groupements terminaux hydroxydiméthylsilyle telles que les huiles de la série 48 V de RHONE POULENC.

Dans cette classe de polydialkylsiloxanes, on peut également mentionner les polyalkylsiloxanes vendus par la société GOLDSCHMIDT sous les dénominations commerciales ABILWAX 9800 et ABILWAX 9801 qui sont des polyalkyl(C₁-C₂₀)siloxanes.

Parmi les polyalkylarylsiloxanes, on peut citer les polydiméthylméthylphénylsiloxanes ou les polydiméthyldiphénylsiloxanes, linéaires ou ramifiés tels que le produit DC 556 COSMETIC GRAD FLUID de DOW CORNING.

Selon l'invention, la silicone de viscosité comprise entre 20 et 38 Pa.s ne comporte pas de fonction amine ou ammonium.

Les compositions capillaires conformes à l'invention renferment les silicones de viscosité convenable définies ci-dessus à des teneurs pondérales qui peuvent être comprises entre 0,05 % et 10 %, de préférence entre 0,1 % et 5 % et encore plus préférentiellement entre 0,2 % et 3%, par rapport au poids total de la composition.

Le véhicule, ou support, des compositions détergentes selon l'invention est de préférence de l'eau ou une solution hydroalcoolique d'un alcool inférieur tel que éthanol, isopropanol ou butanol.

Les compositions détergentes selon l'invention présentent un pH final généralement compris entre 3 et 10. De préférence, ce pH est compris entre 5 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions détergentes selon l'invention peuvent bien entendu contenir en outre tous les adjuvants usuels rencontrés dans le domaine des shampooings, comme par exemple des parfums, des agents conservateurs, des séquestrants, des épaississants, des adoucissants, des modificateurs de mousse, des colorants, des agents nacrants, des agents hydratants, des agents antipelliculaires ou anti-séborrhéiques, des vitamines, des filtres solaires, des agents de mise en suspension et autres.

Bien entendu, l'homme de l'art veillera à choisir ce ou ces éventuels composés complémentaires et/ou leurs quantités de manière telle que les propriétés avantageuses attachées intrinsèquement à l'association quaternaire (base lavante + polymère cationique + deux silicones spécifiques) conforme à l'invention ne soient pas, ou substantiellement pas, altérées par la ou les adjonctions envisagées.

Ces compositions peuvent se présenter sous la forme de liquides plus ou moins épaissis, de crèmes ou de gel et elles conviennent principalement au lavage, au soin et/ou le coiffage des cheveux. Elles peuvent aussi se présenter sous la forme de lotions à rincer.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

L'invention a également pour objet un procédé de lavage et de conditionnement des fibres kératiniques telles que les cheveux consistant à appliquer sur lesdites fibres mouillées une quantité efficace d'une composition telle que définie ci-dessus, puis à effectuer un rinçage à l'eau après un éventuel temps de pause.

Comme indiqué précédemment, les compositions conformes à l'invention confèrent aux cheveux, après rinçage, un remarquable effet coiffant qui se manifeste notamment par une facilité de coiffage et de maintien, ainsi qu'un apport de volume et de légèreté, nettement améliorés.

Un exemple concret, mais nullement limitatif, illustrant l'invention va maintenant être donné.

### EXEMPLE

On a réalisé trois compositions de shampooing, l'une conforme à l'invention (composition A) et les deux autres comparatives (compositions B et C) :

| | A Invention | B Comparatif | C Comparatif |
|---|---|---|---|
| - Lauryléthersulphate de sodium (C12/C14 à 70/30) à 2,2 moles d'oxyde d'éthylène (MA = matière active) | 14 gMA | 14 gMA | 14 gMA |
| - MIRANOL C2M (*) | 4,6 gMA | 4,6 gMA | 4,6 gMA |
| - Polymère cationique (**) | 0,1 g | 0,1 g | 0,1 g |
| - Silicone aminée (***) | 1,05 gMA | - | 1,05 gMA |
| - Silicone < 100 000 cSt (60 000 cSt) (****) | 2 g | 2 g | - |
| - Silicone > 100 000 cSt (300 000 cSt) (*****) | - | - | 2 g |
| - Mélange de 1-hexadécyloxy octadodécanol et d'alcool cétylique | 2,5 g | 2,5 g | 2,5 g |
| - Monoisopropanolamide d'acides de coprah | 0,6 g | 0,6 g | 0,6 g |
| - Acide citrique qs pH | 5,2 | 5,2 | 5,2 |
| - Eau déminéralisée qs | 100 g | 100 g | 100 g |

| | | | |
|---|---|---|---|
| (*)Cocoamidoéthyl (N-hydroxyéthyl, N-carboxyméthyl) glycinate de sodium, vendu par RHONE POULENC | | | |
| (**) : gomme de guar modifiée par du chlorure de 2,3-époxypropyl triméthylammonium vendue sous la dénomination JAGUAR C13 S par la société RHONE POULENC | | | |
| (***) : Amodiméthicone vendue en émulsion cationique à 35% de matière active sous la dénomination FLUID DC 939 par la société DOW CORNING | | | |
| (****) : Polydiméthylsiloxane de viscosité 60 000 cSt vendu par la société DOW CORNING sous la dénomination FLUID DC 200 - 60 000 cSt. | | | |
| (*****) : Polydiméthylsiloxane de viscosité 300 000 cSt vendu par la société WACKER sous la dénomination AK 300.000. | | | |

On effectue un shampooing en appliquant environ 12 g de la composition A sur des cheveux préalablement mouillés. On fait mousser le shampooing puis on rince abondamment à l'eau.

On procède selon le même mode opératoire que ci-dessus avec les compositions comparatives B et C.

Un panel d'experts évalue le démêlage des cheveux mouillés, le démêlage des cheveux séchés, la facilité de mise en forme, la douceur et le lissage des cheveux séchés.

Tous les experts indiquent une amélioration nette de ces propriétés pour les cheveux traités avec la composition A selon l'invention.

## Revendications

1. Composition capillaire détergente et conditionnante, **caractérisée par le fait qu'**elle comprend, dans un milieu cosmétiquement acceptable, (A) une base lavante et (B) un système conditionneur comprenant au moins un polymère cationique et un mélange d'au moins une silicone aminée et d'au moins une silicone insoluble de viscosité comprise entre 20 et 80 Pa.s différente de ladite silicone aminée. La viscosité de ces silicones est mesurée à 25°C selon la norme ASTM 445 Appendice C.

2. Composition selon la revendication 1, **caractérisée par le fait que** ladite base lavante comprend un ou plusieurs tensioactifs choisis parmi des tensioactifs anioniques, amphotères, non ioniques, cationiques et leurs mélanges.

3. Composition selon la revendication 1 ou 2, **caractérisée par le fait que** ladite base lavante est présente à une teneur pondérale comprise entre 4 % et 50 % par rapport au poids total de la composition.

4. Composition selon la revendication 3, **caractérisée par le fait que** ladite teneur est comprise entre 10 % et 35 %.

5. Composition selon la revendication 4, **caractérisée par le fait que** ladite teneur est comprise entre 12 % à 25 %.

6. Composition selon l'une quelconque des revendications précédentes, **caractérisée par le fait que** ledit polymère cationique est présent à une teneur pondérale comprise entre 0,001 % et 10 % par rapport au poids total de la composition.

7. Composition selon la revendication 6, **caractérisée par le fait que** ladite teneur est comprise entre 0,005 % et 5 %.

8. Composition selon la revendication 7, **caractérisée par le fait que** ladite teneur est comprise entre 0,01 % et 3 %.

9. Composition selon l'une quelconque des revendications précédentes, **caractérisée par le fait que** ladite silicone insoluble de viscosité inférieure ou égale à 100 Pa.s est présente à une teneur pondérale comprise entre 0,05 % et 10 % par rapport au poids total de la composition.

10. Composition selon la revendication 9, **caractérisée par le fait que** ladite teneur est comprise entre 0,1 % et 5 %.

11. Composition selon la revendication 10, **caractérisée par le fait que** ladite teneur est comprise entre 0,2 % et 3%.

12. Composition selon l'une quelconque des revendications précédentes, **caractérisée par le fait que** ladite silicone aminée est présente à une teneur pondérale comprise entre 0,05 et 10% par rapport au poids total de la composition.

13. Composition selon la revendication 12, **caractérisée par le fait que** ladite teneur est comprise entre 0,1 et 5 % par rapport au poids total de la composition.

14. Composition selon l'une quelconque des revendications précédentes, **caractérisée par le fait que** ledit polymère cationique est choisi parmi les dérivés d'éther de cellulose quaternaires, les cyclopolymères, les polysaccharides cationiques et leurs mélanges.

15. Composition selon la revendication 14, **caractérisée par le fait que** ledit cyclopolymère est choisi parmi les copolymères du chlorure de diméthyldiallylammonium et d'acrylamide.

16. Composition selon la revendication 14, **caractérisée par le fait que** lesdits dérivés d'éther de cellulose quaternaires sont choisis parmi les hydroxyéthylcelluloses ayant réagi avec un époxyde substitué par un groupement triméthylammonium.

17. Composition selon la revendication 14, **caractérisée par le fait que** lesdits polysaccharides cationiques sont choisis parmi les gommes de guar modifiées par un sel de 2,3-époxypropyl triméthylammonium.

18. Composition selon l'une quelconque des revendications précédentes, **caractérisée par le fait que** la silicone de viscosité comprise entre 20 et 80 Pa.s. est choisie parmi (i) les polydialkylsiloxanes, (ii) les polydiarylsiloxanes et (iii) les polyalkylarylsiloxanes.

19. Composition selon l'une quelconque des revendications précédentes, **caractérisée par le fait que** la silicone aminée est choisie parmi :
(a) les polysiloxanes dénommés dans le dictionnaire CTFA "amodiméthicone" et répondant à la formule : 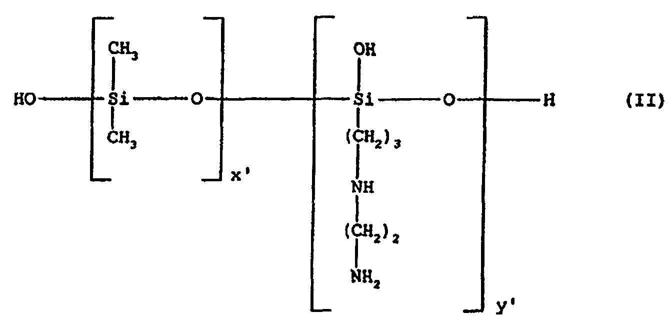 dans laquelle x' et y' sont des nombres entiers dépendant du poids moléculaire, généralement tels que ledit poids moléculaire en nombre est compris entre 5 000 et 500 000 ;
(b) les polymères cationiques siliconés répondant à la formule :
R'ₐG₃₋ₐ-Si(OSiG₂)ₙ-(OSiG_{b}R'_{2-b})ₘ-O-SiG₃₋ₐ-R'ₐ (III)
dans laquelle :
G est un atome d'hydrogène, ou un groupement phényle, OH, ou alkyle en C₁-C₈, par exemple méthyle,
a désigne le nombre 0 ou un nombre entier de 1 à 3, en particulier 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R' est un radical monovalent de formule -C_{q}H_{2q}L dans laquelle q est un nombre de 2 à 8 et L est un groupement aminé éventuellement quatemisé choisi parmi les groupements
-NR"-CH₂-CH₂-N'(R")₂
-N(R")₂
-N^{⊕}(R")₃A⁻
-N^{⊕}H(R")₂A⁻
-N^{⊕}H₂(R")A⁻
-N(R")-CH₂-CH₂-N^{⊕}R"H₂A⁻,
dans lesquels R" peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure ;
(c) les polymères cationiques siliconés répondant à la formule : 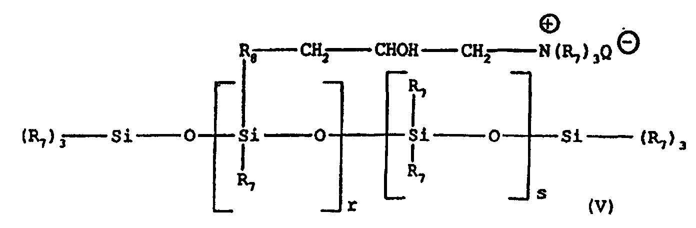
dans laquelle :
R₇ représente un radical hydrocarboné monovalent ayant de 1 à 18 atomes de carbone, et en particulier un radical alkyle en C₁-C₁₈, ou alcényle en C₂-C₁₈, par exemple méthyle ;
R₈ représente un radical hydrocarboné divalent, notamment un radical alkylène en C₁-C₁₈ ou un radical alkylèneoxy divalent en C₁-C₁₈, par exemple en C₁-C₈ ;
Q⁻ est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

20. Composition selon l'une quelconque des revendications précédentes, **caractérisée par le fait qu'**elle présente un pH compris entre 3 et 10.

21. Composition selon l'une quelconque des revendications précédentes, **caractérisée en ce que** ledit système conditionneur est exempt de silicones autres que des silicones aminées et des silicones insolubles de viscosité comprise entre 20 et 80 Pa.s.

22. Utilisation d'une composition telle définie à l'une quelconque des revendications précédentes pour le nettoyage et/ou le conditionnement des cheveux.

23. Procédé de lavage et de conditionnement des fibres kératiniques telles que les cheveux consistant à appliquer sur lesdites fibres mouillées une quantité efficace d'une composition telle que définie à l'une quelconque des revendications 1 à 22, puis à effectuer un rinçage à l'eau après un éventuel temps de pause.

## Claims

1. Detergent and conditioning hair-care composition, **characterized in that** it comprises, in a cosmetically acceptable medium, (A) a washing base and (B) a conditioning system comprising at least one cationic polymer and a mixture of at least one aminated silicone and at least one insoluble silicone of viscosity between 20 Pa.s and 80 Pa.s which is different from the said aminated silicone. The, viscosity of these silicones is measured at 25°C according to ASTM Standard 445 Appendix C.

2. Composition according to Claim 1, **characterized in that** the said washing base comprises one or more surfactants chosen from anionic, amphoteric, nonionic and cationic surfactants and mixtures thereof.

3. Composition according to Claim 1 or 2, **characterized in that** the said washing base is present in a weight content of between 4 % and 50 % relative to the total weight of the composition.

4. Composition according to Claim 3, **characterized in that** the said content is between 10 % and 35 %.

5. Composition according to Claim 4, **characterized in that** the said content is between 12 % and 25 %.

6. Composition according to any one of the preceding claims, **characterized in that** the said cationic polymer is present in a weight content of between 0.001 % and 10 % relative to the total weight of the composition.

7. Composition according to Claim 6, **characterized in that** the said content is between 0.005 % and 5 %.

8. Composition according to Claim 7, **characterized in that** the said content is between 0.01 % and 3 %.

9. Composition according to any one of the preceding claims, **characterized in that** the said insoluble silicone of viscosity between 20 and 80 Pa.s is present in a weight content of between 0.05 % and 10 % relative to the total weight of the composition.

10. Composition according to Claim 9, **characterized in that** the said content is between 0.1 % and 5 %.

11. Composition according to Claim 10, **characterized in that** the said content is between 0.2 % and 3 %.

12. Composition according to any one of the preceding claims, **characterized in that** the said aminated silicone is present in a weight content of between 0.05 and 10 % relative to the total weight of the composition.

13. Composition according to Claim 12, **characterized in that** the said content is between 0.1 and 5 % relative to the total weight of the composition.

14. Composition according to any one of the preceding claims, **characterized in that** the said cationic polymer is chosen from quaternary cellulose ether derivatives, cyclopolymers, cationic polysaccharides and mixtures thereof.

15. Composition according to Claim 14, **characterized in that** the said cyclopolymer is chosen from copolymers of dimethyldiallylammonium chloride and acrylamide.

16. Composition according to Claim 14, **characterized in that** the said quaternary cellulose ether derivatives are chosen from hydroxyethylcelluloses which have reacted with an epoxide substituted with a trimethylammonium group.

17. Composition according to Claim 14, **characterized in that** the said cationic polysaccharides are chosen from guar gums modified with a 2,3-epoxypropyltrimethylammonium salt.

18. Composition according to any one of the preceding claims, **characterized in that** the silicone of viscosity between 20 and 80 Pa.s is chosen from (i) polydialkylsiloxanes, (ii) polydiarylsiloxanes and (iii) polyalkylarylsiloxanes.

19. Composition according to any one of the preceding claims, **characterized in that** the aminated silicone is chosen from:
(a) the polysiloxanes named in the CTFA Dictionary "amodimethicone" and corresponding to the formula; 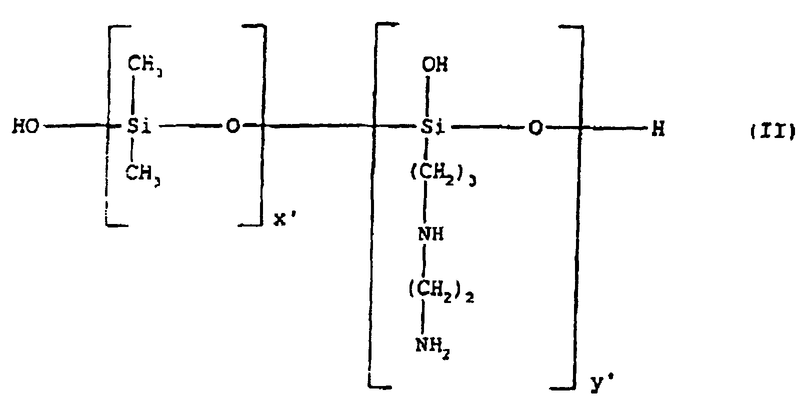 in which x' and y' are integers depending on the molecular weight, generally such that the said number-average molecular weight is between 5,000 and 500,000;
(b) the cationic silicone polymers corresponding to the formula:
R'ₐG₃₋ₐ-Si(OSiG₂)ₙ-(OSiG_{b}R'_{2-b})ₘ-O-SiG₃₋ₐ-R'ₐ (III)
in which:
G is a hydrogen atom or a phenyl, OH or C₁-C₈ alkyl group, for example methyl,
a denotes the number 0 or an integer from 1 to 3, especially 0,
b denotes 0 or 1, and especially 1,
m and n are numbers such that the sum (n + m) can vary, in particular, from 1 to 2,000, and
especially from 50 to 150, it being possible for n to denote a number from 0 to 1,999 and in particular from 49 to 149 and for m to denote a number from 1 to 2,000 and in particular from 1 to 10;
R' is a monovalent radical of formula -C_{q}H_{2q}L in which q is a number from 2 to 8 and L is an optionally quaternized amino group chosen from the groups:
-NR"-CH₂-CH₂-N'(R")₂
-N(R")₂
-N⁺(R")₃A⁻
-N⁺(R")₂A⁻
-N⁺H₂(R")A⁻
-N(R") -CH₂-CH₂-N⁺R"H₂A⁻,
in which R" can denote hydrogen, phenyl, benzyl or a saturated monovalent hydrocarbon radical, for example an alkyl radical having from 1 to 20 carbon atoms, and A⁻ represents a halide ion such as, for example, fluoride, chloride, bromide or iodide;
(c)the cationic silicone polymers corresponding to the formula: 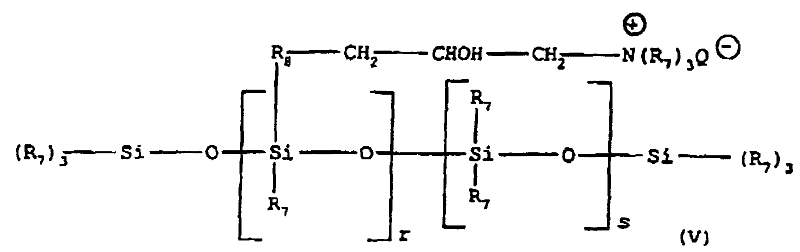 in which:
R₇ represents a monovalent hydrocarbon radical having from 1 to 18 carbon atoms, and especially a C₁-C₁₈ alkyl or C₂-C₁₈ alkenyl radical, for example methyl;
R₈ represents a divalent hydrocarbon radical, in particular a C₁-C₁₈ alkylene radical or a divalent C₁-C₁₈, for example C₁-C₈, alkyleneoxy radical;
Q⁻ is a halide ion, in particular chloride;
r represents an average statistical value from 2 to 20, and especially from 2 to 8;
s represents an average statistical value from 20 to 200, and especially from 20 to 50.

20. Composition according to any one of the preceding claims, **characterized in that** it has a pH of between 3 and 10.

21. Composition according to any one of the preceding claims, **characterized in that** the said conditioning system is free from silicones other than aminated silicones and insoluble silicones of viscosity between 20 and 80 Pa.s.

22. Use of a composition as defined in any one of the preceding claims, for cleansing and/or conditioning the hair.

23. Process for washing and conditioning keratinous fibres such as the hair, consisting in applying to the said fibres in the wet state an effective amount of a composition as defined in any one of Claims 1 to 21, and in then rinsing with water after an optional period of exposure.

## Patentansprüche

1. Reinigende und konditionierende Zusammensetzung für das Haar, **dadurch gekennzeichnet, daß** sie in einem kosmetisch akzeptablen Medium (A) eine reinigende Basisformulierung und (B) ein Konditioniersystem enthält, das mindestens ein kationisches Polymer und ein Gemisch von mindestens einem aminierten Silicon und mindestens einem unlöslichen Silicon mit einer Viskosität von 20 bis 80 Pa·s enthält, das von dem aminierten Silicon verschieden ist, wobei die Viskosität bei 25 °C nach der Norm ASTM 445 Anhang C bestimmt wird.

2. Zusammensetzung nach Anspruch 1, **dadurch gekennzeichnet, daß** die reinigende Basisformulierung einen oder mehrere grenzflächenaktive Stoffe enthält, die unter den anionischen, amphoteren, nichtionischen und kationischen grenzflächenaktiven Stoffen und deren Gemischen ausgewählt sind.

3. Zusammensetzung nach Anspruch 1 oder 2, **dadurch gekennzeichnet, daß** die reinigende Basisformulierung in einem Gewichtsanteil von 4 bis 50 %, bezogen auf das Gesamtgewicht der Zusammensetzung, vorliegt.

4. Zusammensetzung nach Anspruch 3, **dadurch gekennzeichnet, daß** der Gewichtsanteil im Bereich von 10 bis 35 % liegt.

5. Zusammensetzung nach Anspruch 4, **dadurch gekennzeichnet, daß** der Gewichtsanteil im Bereich von 12 bis 25 % liegt.

6. Zusammensetzung nach einem der vorhergehenden Ansprüche, **dadurch gekennzeichnet, daß** das kationische Polymer in einem Gewichtsanteil von 0,001 bis 10 %, bezogen auf das Gesamtgewicht der Zusammensetzung, vorliegt.

7. Zusammensetzung nach Anspruch 6, **dadurch gekennzeichnet, daß** der Gewichtsanteil im Bereich von 0,005 bis 5 % liegt.

8. Zusammensetzung nach Anspruch 7, **dadurch gekennzeichnet, daß** der Gewichtsanteil im Bereich von 0,01 bis 3 % liegt.

9. Zusammensetzung nach einem der vorhergehenden Ansprüche, **dadurch gekennzeichnet, daß** das unlösliche Silicon mit einer Viskosität von höchstens 100 Pa·s in einem Gewichtsanteil von 0,05 bis 10 %, bezogen auf das Gesamtgewicht der Zusammensetzung, vorliegt.

10. Zusammensetzung nach Anspruch 9, **dadurch gekennzeichnet, daß** der Gewichtsanteil im Bereich von 0,1 bis 5 % liegt.

11. Zusammensetzung nach Anspruch 10, **dadurch gekennzeichnet, daß** der Gewichtsanteil im Bereich von 0,2 bis 3 % liegt.

12. Zusammensetzung nach einem der vorhergehenden Ansprüche, **dadurch gekennzeichnet, daß** das aminierte Silicon in einem Gewichtsanteil von 0,05 bis 10 %, bezogen auf das Gesamtgewicht der Zusammensetzung, vorliegt.

13. Zusammensetzung nach Anspruch 12, **dadurch gekennzeichnet, daß** der Gewichtsanteil im Bereich von 0,1 bis 5 %, bezogen auf das Gesamtgewicht der Zusammensetzung, liegt.

14. Zusammensetzung nach einem der vorhergehenden Ansprüche, **dadurch gekennzeichnet, daß** das kationische Polymer unter den quartären Celluloseetherderivaten, Cyclopolymeren, kationischen Polysacchariden und deren Gemischen ausgewählt ist.

15. Zusammensetzung nach Anspruch 14, **dadurch gekennzeichnet, daß** das Cyclopolymer unter den Copolymeren von Dimethyldiallylammoniumchlorid und Acrylamid ausgewählt ist.

16. Zusammensetzung nach Anspruch 14, **dadurch gekennzeichnet, daß** die quartären Celluloseetherderivate unter den Hydroxyethylcellulosen ausgewählt ist, die mit einem mit einer Trimethylammoniumgruppe substituierten Epoxid umgesetzt wurden.

17. Zusammensetzung nach Anspruch 14, **dadurch gekennzeichnet, daß** die kationischen Polysaccharide unter den mit einem 2,3-Epoxypropyltrimethylammoniumsalz modifizierten Guargummen ausgewählt sind.

18. Zusammensetzung nach einem der vorhergehenden Ansprüche, **dadurch gekennzeichnet, daß** das Silicon mit einer Viskosität von 20 bis 80 Pa·s unter (i) den Polydialkylsiloxanen, (ii) den Polydiarylsiloxanen und (iii) den Polyalkylarylsiloxanen ausgewählt ist.

19. Zusammensetzung nach einem der vorhergehenden Ansprüche, **dadurch gekennzeichnet, daß** das aminierte Silicon ausgewählt ist unter:
(a) Polysiloxanen, die gemäß CTFA-Nomenklatur als "Amodimethicone" bezeichnet werden und der folgenden Formel entsprechen: 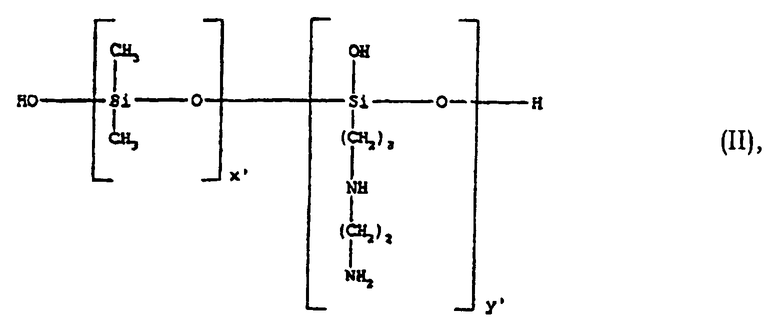 worin x' und y' ganze Zahlen bedeuten, die von der Molmasse abhängen und im allgemeinen so gewählt sind, daß das Zahlenmittel des Molekulargewichts im Bereich von etwa 5 000 bis 500 000 liegt;
(b) kationische Siliconpolymere der folgenden Formel:
R'ₐG₃₋ₐ-Si(OSiG₂)ₙ-(OSiG_{b}R'_{2-b})ₘ-O-SiG₃₋ₐ-R'ₐ (III),
worin bedeuten:
G Wasserstoff, Phenyl, OH, C₁₋₈-Alkyl, beispielsweise Methyl,
a 0 oder eine ganze Zahl von 1 bis 3 und insbesondere 0,
b 0 oder 1 und insbesondere 1,
m und n Zahlen, die so ausgewählt sind, daß die Summe (n + m) insbesondere im Bereich von 1 bis 2000 und besonders 50 bis 150 liegen kann, wobei n eine Zahl von 0 bis 1999 und insbesondere 49 bis 149 und m eine Zahl von 1 bis 2000 und insbesondere 1 bis 10 bedeuten kann,
die Gruppe R' eine einwertige Gruppe der Formel -C_{q}H_{2q}L, worin q eine Zahl von 1 bis 8 ist und L eine gegebenenfalls quatemisierte aminierte Gruppe bedeutet, welche unter den folgenden Gruppen ausgewählt ist:
-NR"-CH₂-CH₂-N'(R")₂
-N(R")₂
-N^{⊕}(R")₃A⁻
-N^{⊕}H(R")₂A⁻
-N^{⊕}H₂(R")A⁻
-N(R")-CH₂-CH₂-N^{⊕}R"H₂A⁻,
worin R" Wasserstoff, Phenyl, Benzyl oder eine einwertige gesättigte Kohlenwasserstoffgruppe, beispielsweise eine Alkylgruppe mit 1 bis 20 Kohlenstoffatomen bedeuten kann und A-ein Halogenid bedeutet, wie beispielsweise Fluorid, Chlorid, Bromid oder Iodid;
(c) kationische Siliconpolymere der folgenden Formel: 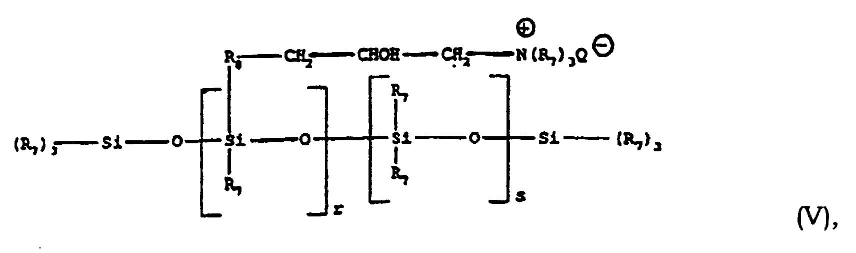 worin bedeuten:
R₇ eine einwertige Kohlenwasserstoffgruppe mit 1 bis 18 Kohlenstoffatomen und insbesondere C₁₋₁₈-Alkyl oder C₂₋₁₈-Alkenyl, beispielsweise Methyl;
R₈ eine zweiwertige Kohlenwasserstoffgruppe, insbesondere eine C₁₋₁₈-Alkylengruppe oder eine zweiwertige C₁₋₁₈-Alkylenoxygruppe, beispielsweise eine Gruppe mit 1 bis 8 Kohlenstoffatomen;
Q⁻ ein Halogenid, insbesondere Chlorid;
r einen statistischen Mittelwert von 2 bis 20 und insbesondere 2 bis 8; und
s einen statistischen Mittelwert von 20 bis 200 und insbesondere 20 bis 50.

20. Zusammensetzung nach einem der vorhergehenden Ansprüche, **dadurch gekennzeichnet, daß** sie einen pH-Wert im Bereich von 3 bis 10 aufweist.

21. Zusammensetzung nach einem der vorhergehenden Ansprüche, **dadurch gekennzeichnet, daß** das Konditioniersystem neben den aminierten Siliconen und den unlöslichen Siliconen mit einer Viskosität von 20 bis 80 Pa·s keine weiteren Silicone enthält

22. Verwendung einer Zusammensetzung nach einem der vorhergehenden Ansprüche zur Reinigung und/oder zum Konditionieren der Haare.

23. Verfahren zum Waschen und Konditionieren von Keratinfasern, wie dem Haar, das darin besteht, auf die feuchten Fasern eine wirksame Menge einer Zusammensetzung nach einem der Ansprüche 1 bis 22 aufzutragen und anschließend, gegebenenfalls nach einer Einwirkzeit, mit Wasser zu spülen.
